# EUROPEAN PATENT SPECIFICATION

(11) **EP 2 234 644 B1**
(45) Date of publication and mention of the grant of the patent: **31.07.2013**
(21) Application number: 09700220.8
(22) Date of filing: 05.01.2009
(51) Int. Cl.: A61K 47/46, A61K 38/28

(54) **INSULIN FORMULATIONS FOR INSULIN RELEASE AS A FUNCTION OF TISSUE GLUCOSE LEVELS**
INSULINFORMULIERUNGEN ZUR INSULINFREISETZUNG ALS FUNKTION DES GEWEBEGLUKOSESPIEGELS
PRÉPARATIONS D'INSULINE POUR LIBÉRATION D'INSULINE EN FONCTION DE NIVEAUX DE GLUCOSE DANS LES TISSUS

(30) Priority: 04.01.2008 US 19187
(43) Date of publication of application: 06.10.2010
(73) Proprietor: Biodel, Inc., Danbury, CT 06810 (US)
(72) Inventor: KASHYAP, Nandini, Danbury, CT 06810 (US); STEINER, Solomon, S., Mount Kisco, NY 10549 (US); POHL, Roderike, Sherman, CT 06784 (US)
(74) Representative: Wright, Andrew John
(86) International application number: PCT/US2009/030153
(87) International publication number: WO 2009/089181

(56) References cited:
- WO-A-2006/088473
- TRAITEL T ET AL: "Characterization of glucose-sensitive insulin release systems in simulated in vivo conditions" BIOMATERIALS, ELSEVIER SCIENCE PUBLISHERS BV., BARKING, GB, vol. 21, no. 16, 1 August 2000 (2000-08-01), pages 1679-1687, XP004200587 ISSN: 0142-9612
- KASHYAP ET AL: "Design and evaluation of biodegradable, biosensitive in situ gelling system for pulsatile delivery of insulin" BIOMATERIALS, ELSEVIER SCIENCE PUBLISHERS BV., BARKING, GB, vol. 28, no. 11, 31 January 2007 (2007-01-31), pages 2051-2060, XP005879814 ISSN: 0142-9612
- ZHANG K ET AL: "Modulated insulin permeation across a glucose-sensitive polymeric composite membrane" JOURNAL OF CONTROLLED RELEASE, ELSEVIER, AMSTERDAM, NL, vol. 80, no. 1-3, 23 April 2002 (2002-04-23), pages 169-178, XP004348633 ISSN: 0168-3659

## Description

### FIELD OF THE INVENTION

The present invention generally relates to formulations containing insulin and a glucose oxidizing agent and/or an enzyme for the treatment of diabetes.

### BACKGROUND OF THE INVENTION

Glucose is a simple sugar used by all the cells of the body to produce energy and support life. Humans need a minimum level of glucose in their blood at all times to stay alive. The primary manner in which the body produces blood glucose is through the digestion of food. When a person is not getting this glucose from food digestion, glucose is produced from stores in the tissue and released by the liver. The body's glucose levels are regulated by insulin. Insulin is a peptide hormone that is naturally secreted by the pancreas. Insulin helps glucose enter the body's cells to provide a vital source of energy.

When a healthy individual begins a meal, the pancreas releases a natural spike of insulin called the first-phase insulin release. In addition to providing sufficient insulin to process the glucose coming into the blood from digestion of the meal, the first-phase insulin release acts as a signal to the liver to stop making glucose while digestion of the meal is taking place. Because the liver is not producing glucose and there is sufficient additional insulin to process the glucose from digestion, the blood glucose levels of healthy individuals remain relatively constant and their blood glucose levels do not become too high.

Diabetes is a disease characterized by abnormally high levels of blood glucose and inadequate levels of insulin. There are two major types of diabetes, *i.e.* Type 1 and Type 2. In Type 1 diabetes, the body produces no insulin. In the early stages of Type 2 diabetes, although the pancreas does produce insulin, either the body does not produce the insulin at the right time or the body's cells ignore the insulin, a condition known as insulin resistance.

Hyperglycemia is a condition in which an excessive amount of glucose circulates in an individual's blood plasma. This condition generally results when a patient has a blood glucose level of 10 mmol/L (180 mg/dl) or greater, but symptoms and effects may not start to become noticeable until greater blood glucose concentrations are reached, such as 15 to 20 mmol/L, (270 to 360 mg/dl) or greater. Hyperglycemia causes glucose to attach unnaturally to certain proteins in the blood, interfering with the proteins' ability to perform their normal function of maintaining the integrity of the small blood vessels. With hyperglycemia occurring after each meal; the tiny blood vessels eventually break down and leak. The long-term adverse effects of hyperglycemia include blindness, loss of kidney function, nerve damage and loss of sensation and poor circulation in the periphery, potentially requiring amputation of the extremities.

Because patients with Type 1 diabetes produce no insulin, the primary treatment for Type 1 diabetes is multiple daily insulin injection therapy, referred to as "intensive insulin treatment". The treatment of Type 2 diabetes typically starts with management of diet and exercise. Although helpful in the short-run, treatment through diet and exercise alone is not an effective long-term solution for the majority of patients with Type 2 diabetes. When diet and exercise are no longer an effective means for maintaining safe blood glucose levels, treatment often commences with various non-insulin oral medications. These oral medications act by increasing the amount of insulin produced by the pancreas, by increasing the sensitivity of insulin-sensitive cells, by reducing the glucose output of the liver or by some combination of these mechanisms. These treatments are limited in their ability to manage the disease effectively and generally have significant side effects, such as weight gain and hypertension. Because of the limitations of non-insulin treatments, many patients with Type 2 diabetes deteriorate over time and eventually require insulin therapy to support their metabolism. Patients with Type 2 diabetes who still produce some insulin on their own are often characterized as patients who are "not fully insulin dependent."

Insulin therapy has been used for more than 80 years to treat diabetes. Intensive insulin therapy for diabetes involves providing a basal insulin, ideally present at a uniform level in the blood over a 24-hour period and a bolus or meal time (prandial) insulin to cover the added carbohydrate load from digestion concomitant with each meal. This therapy usually involves administering several injections of insulin each day. These injections consist of administering a long-acting basal injection one or two times per day and an injection of a fast acting insulin at meal-time, *i*.*e*. a prandial insulin. Although this treatment regimen is accepted as effective, it has limitations. First, patients generally dislike injecting themselves with insulin due to the inconvenience and pain of needles. As a result, patients tend not to comply adequately with the prescribed treatment regimens and are often improperly medicated.

In many places, basal insulin is provided by the administration of two daily doses of NPH insulin, separated by 12 hours. Neutral Protamine Hagedorn ("NPH") insulin is a suspension of crystalline zinc insulin combined with the positively charged polypeptide, protamine, at pH 7. NPH insulin has the advantage that it can be mixed with an insulin that is released more quickly than NPH insulin, which compliments NPH insulin's relatively long lasting action compared to an insulin, *e.g*. human recombinant insulin, adminstered alone.

A patient eating three meals a day and using NPH insulin as the basal insulin requires five injections per day, one with each of three meals and two NPH insulin injections, one in the morning and the other at bedtime. To reduce the number of injections the patient must take, the morning dose of NPH insulin has been combined with a short acting insulin, such as recombinant human insulin, or a rapid acting insulin analog, such as insulin lispro. A typical combination is a 70% NPH to 30% rapid acting insulin analog mixture. As a result, the patient can reduce the number of injections from five per day to four per day. *See, e.g,* Garber, Drugs, 66(1):31-49 (2006).

Insulin glargine, which is currently sold under the trade name LANTUS® (Sanofi-Aventis Deutschland GmbH), is marketed as a "long-acting" insulin analog. LANTUS® can have up to a 24-hour duration. LANTUS® typically starts to lower blood glucose about one hour after injection. J. Rosenstock and colleagues found that patients who took insulin glargine had a much lower risk of low blood glucose (hypoglycemia) than the patients who took NPH insulin. While LANTUS® is designed to cover the average patient's basal insulin needs over a 24-hour time period, the reality is that for many patients, it does not last long enough, causing them to be hyperglycemic, typically in the early morning hours. Additionally, LANTUS® does not adjust the amount of insulin released from the formulation based on the patient's needs. Thus it may release more or less insulin than a patient needs to cover the patient's needs at a given time period.

Prandial insulins, such as rapid-acting insulin treatments include insulin analogs, such as insulin lispro (sold by Eli Lilly® as HUMALOG®), insulin glulisine (sold by Sanofi-Aventis® as APIDRA®) and insulin aspart (sold by Novo Nordisk® as NOVOLOG®). However, for the rapid-acting insulin analogs, a patient's insulin analog levels are based on the time and the quantity of insulin injected (with peak insulin levels typically occurring within 50 to 70 minutes following the injection) and peak plasma levels of the rapid acting analogs are independent of the tissue glucose levels.

Current prandial insulins do not respond to increased blood glucose levels in a patient; thus if a patient underestimates his/her blood glucose levels due to eating a meal, current prandial insulin formulations are not able regulate the patient's blood glucose levels. And the patient may become hyperglycemic.

Because the rapid-acting insulin analogs do not adequately mimic the feedback mechanism of the first-phase insulin release of a non-diabetic individual, patients with diabetes using insulin therapy continue to have inadequate levels of insulin present at the initiation of a meal and too much insulin present between meals. This lag in insulin delivery can result in hyperglycemia early after meal onset. Furthermore, the excessive insulin between meals may result in an abnormally low level of blood glucose known as hypoglycemia. Hypoglycemia can result in loss of mental acuity, confusion, increased heart rate, hunger, sweating and faintness. At very low glucose levels, such as below 60 mg/dl, hypoglycemia can result in loss of consciousness, coma and even death. According to the American Diabetes Association ("ADA"), insulin-using diabetic patients have on average 1.2 serious hypoglycemic events per year, many of which events require hospital emergency room visits by the patients.

Even when insulin injections are properly administered, they do not replicate the natural glucose feedback profile of insulin. Injected insulin enters the blood slowly, with no regard to the current blood glucose level. A limitation to the currently administered basal therapies is that there is no feedback mechanism to determine the amount of insulin that is released based on the blood glucose levels. In particular, there is a need to mimic the natural feedback that allows blood insulin levels to rise in response to an increase in glucose levels that occurs in a person *without* diabetes. The problem with the existing basal insulin treatments is that they are insensitive to the daily variance in a patient's diet, exercise, stress and numerous other factors which result in fluctuations of the blood glucose levels.

Hydrogels have been used to develop a feedback mechanism based on glucose sensitivity for "smart" drug delivery systems, *i.e.* a system that delivers drug based on a patient's needs. Glucose sensitive hydrogels have been used to control insulin release by changing the gel structure in response to environmental glucose concentrations.

One of the initial studies in this direction was the use of a lectin, Concanavalin-A (ConA). This approach was based on competitive binding, where the glycosylated insulin molecule is bound to each subunit of ConA and is reversibly replaced from it by glucose in direct proportion to external glucose concentration. This system suffers from the drawback that ConA is immunogenic and glycosylation of insulin makes it a new chemical entity. ConA has significant toxicity issues, and there is a significant risk that a patient could develop antibodies against ConA. Because of this risk, it is doubtful that such a product could ever gain regulatory approval. Therefore very expensive and extensive testing of a formulation ConA would be required before it could even be considered for approval for treatment of humans.

In another approach, polymers having pendant phenyl boronic acids have been used as a crosslinking agent enabling gel formation with a polyol (such as poly-vinyl alcohol) to form a glucose sensitive gels. Kitano, J. Con. Rel. 19:162-170(1992). Boronic acids are known to bind to free hydroxyl groups with an affinity for diols (including monosaccharide molecules such as glucose and fructose). In the presence of glucose, the boronic acid-containing gel swells due to the substitution reaction of the vinyl alcohols with the free glucose. The swelling of the hydrogel results in the release of insulin that was previously trapped in the crosslinked polymer network. The major limitations of this system include that boronic acids are only sensitive to glucose under alkaline conditions (pH>9). In addition, boronic acids are less selective for glucose over other monosaccarides.

Another glucose sensitive polymeric hydrogel contains glucose dehydrogenase (GDH). *See* Chung, et al., J. Con Rel. 18:45-54 (1992). In this system insulin is grafted onto the polymer surface with disulfide linkages. When GDH is exposed to glucose, GDH oxidizes glucose molecules to release electrons. The released electrons, reduce the disulfide bond for the release of grafted insulin. GDH system accomplishes insulin release by using various enzyme cofactors acting as an electron mediator that also need to be grafted onto the polymer. This system can provide improved sensitivity to glucose. However a major drawback of GDH based system is the limited amount of insulin that can be grafted onto the polymer surface. As a result, it is doubtful that sufficient amounts of insulin to produce a clinically useful effect could be employed in this system in a volume that is sufficiently small to be useful as a subcutaneous injection.

Glucose Oxidase has been immobilized onto pH-sensitive hydrogels. *See* Podual, J. Con. Rel. 67:9-17(1999); Polymer 41:3975-3983 (2000). The conversion of glucose to gluconic acid, catalyzed by glucose oxidase, lowers the pH affecting the swelling of pH sensitive hydrogels. This swelling allows a release of insulin in response to an increase in glucose concentrations in the immediate environment. This concept again has limitations as it requires a highly pH-sensitive polymer. All glucose sensitive hydrogels also have the additional limitation concerning the rate of diffusion of insulin out of the polymeric network.

A biodegradable glucose-sensitive in situ gelling system based on chitosan and further comprising glucose oxidase, peroxidase and Insulin was developed. *See* Kashyap, et al., Biomaterials Vol. 28, No. 11, 2051-2060 (2007)

To effectively control diabetes and prevent hypoglycemic complications, it is most desirable to administer insulin in a manner that precisely matches the physiological needs at any given moment. Because the variance in the blood glucose levels is dependent on so many factors, there is a significant need for insulin that can become physiologically available as a result of changes in the body's glucose levels.

Therefore, it is an object of the invention to provide an improved insulin formulation.

It is a further object of the invention to provide an improved method for regulating blood glucose levels in patients in need of insulin treatments, including patients with Type 2 and Type 1 diabetes.

It is a further object of the invention to provide methods for forming improved insulin formulations.

### SUMMARY OF THE INVENTION

Sterile injectable insulin formulations, which are solutions, and that are capable of modifying the amount of insulin released based on the patient's tissue glucose levels, methods for making and using these formulations are described herein. The formulation may be administered via subcutaneous, intradermal or intramuscular administration. In one preferred embodiment, the formulations are administered via subcutaneous injection. The sterile injectable formulations are solutions, and contain insulin, a glucose oxidase and a peroxidase a diluent, and the pH of the formulation is below the isoelectric point of the insulin. Preferably the formulation contains an insulin, a diluent, glucose oxidase and peroxidase. If a thickening agent is present in the formulation, the thickening agent increases the viscosity of the formulation following administration. Following administration to a patient, the insulin is released from the formulations as a function of the patient's tissue glucose level, which in turn maintains the patient's blood glucose level within an optimum range. The formulation is often referred to as a "smart" formulation since it modifies its release rate of insulin according to the patient's needs at a particular time.

In a preferred embodiment, the formulation is designed to release insulin into the systemic circulation over time with a basal release profile following injection in a patient. In another embodiment, the formulation is designed to release insulin into the systemic circulation over time with a non-basal release profile following injection in a patient, such as a regular human insulin release profile or a prandial release profile.

As a patient's blood glucose levels rise, the glucose is oxidized by GOD, resulting in production of hydrogen ions in the microenvironment of the formulation at injection site. The increase in hydrogen ion production will lower the pH of the microenvironment below the isoelectric point for the insulin, making the insulin more soluble and releasing it into systemic circulation. Availability of insulin in systemic circulation leads to decreased blood glucose levels. Following this decrease in blood glucose levels, the reaction that converts glucose to gluconic acid slows down. Thereby decreasing the production of hydrogen ions, and increasing the pH of the microenvironment. This change in pH provides a less soluble environment for the insulin.

When high glucose concentrations, such as 150mg/dl or above, are present in a patient's blood, there is generation of gluconic acid from the oxidation of glucose by the oxidizing agent and/or an enzyme in the formulation. This, in turn, leads to higher release of insulin from the formulation. When the patient's blood glucose concentrations decrease, such as to 80mg/dl or lower, decreased amounts of glucose are present for the oxidizing agent and/or an enzyme to carry out reaction that converts glucose to gluconic acid. Thereby decreasing the production of hydrogen ions, and increasing the pH of the microenvironment, as described above. This change in pH provides a less soluble environment for the insulin, and less insulin is released from the formulation than was released at the lower pH.

### BRIEF DESCRIPTION OF THE DRAWINGS

Figure 1 is a graph of the insulin concentration (mg/mL) in the presence and absence of glucose from a smart basal formulation *in vitro* over time (hours). Set 1 is media with glucose. Set 2 is media without glucose. A, B and C are triplicate runs.
Figure 2 is a bar graph of the mean amount of insulin (mg) released in the presence and absence of glucose from a smart basal formulation *in vitro* (n = 3).
Figure 3 is a bar graph of the amount of insulin released (mg) at different glucose concentrations at two different time intervals, three hours and 6 hours. Concentrations of glucose tested were 0 (empty bar), 100 (grey bar between the empty bar and the black bar), 200 (black bar), 250 (grey bar between the black bar and bar with diagonal lines), 300 (bar with diagonal lines) mg/dl.
Figure 4 is a graph of mean plasma glucose levels (mg/dl) of test and control groups in diabetic swine versus time (minutes). The test group received the smart basal formulation described in Example 1, while the control group received insulin glargine (LANTUS®) (Time = -0 to 1440 minutes, n=3, mean +/-SEM).
Figure 5 is a graph of mean plasma glucose levels (mg/dl) of test and control groups in diabetic swine before feeding versus time (minutes). Figure 5 corresponds with the portion of Figure 4 from time = -25 minutes to time = 250 minutes.
Figure 6 is a graph of mean plasma glucose levels (mg/dl) of test and control groups in diabetic swine after feeding versus time (minutes). Figure 6 corresponds with the portion of Figure 4 from time = 300 minutes to time = 800 minutes.
Figure 7 is a graph of mean plasma insulin levels (µU/ml) (+/-SEM) of test (N=5) and control groups (N=7) in diabetic swine versus time (minutes). The test group received the smart basal formulation described in Example 1, while control group received insulin glargine (LANTUS®).
Figure 8 is a graph of mean plasma glucose levels (mg/dl) (+/-SEM) of test (N=5) and control groups (N=7) in diabetic swine versus time (minutes). The test group received the smart basal formulation described in Example 1, while control group received insulin glargine (LANTUS®).

### DETAILED DESCRIPTION OF THE INVENTION

### I. Definitions

As used herein, "a less soluble insulin" refers to an insulin or insulin analog that is less soluble than recombinant human insulin in extracellular fluid, such as Earle's balanced salt solution E2888 (Sigma Aldrich) at physiological pH (6.2-7.4) and body temperature (*e.g*. 37° C).

As used herein, "a basal insulin" refers to an insulin or insulin formulation that provides prolonged levels of insulin over a period of time after administration of about 12 to 24 hours and that delivers an effective amount of insulin to a patient to manage the patient's normal daily blood glucose fluctuations in the absence of a meal.

As used herein, "a basal release profile" refers to the amount and rate of release of insulin from the formulation into a patient's systemic circulation. In a graph of the patient's mean plasma insulin levels over time, a basal release profile generally has a minimal peak (often referred to as "a peakless profile") and slowly and continuously releases insulin for a prolonged period of time, such as twelve to twenty-four hours following administration. One example of a formulation with a basal release profile is LANTUS®.

As used herein "a non-basal release profile" refers to the amount and rate of release of insulin from the formulation into a patient's systemic circulation. A non-basal release profile has a peak in a graph of the patient's mean plasma insulin levels over time.

As used herein "a regular human insulin release profile" refers to the amount and rate of release of insulin from the formulation into a patient's systemic circulation. In a graph of the patient's mean plasma insulin levels over time, a regular human insulin release profile reaches its peak in within about two hours following injection. One example of a formulation with a regular human insulin release profile is HUMULIN® R.

As used herein "a prandial release profile" refers to the amount and rate of release of insulin from the formulation into a patient's systemic circulation. In a graph of the patient's mean plasma insulin levels over time, a prandial release profile generally has a rapid release of insulin following injection, which reaches its peak in about one hour or less. One example of a formulation with a prandial release profile is VIAJECT™.

As used herein, "a prandial insulin" refers to an insulin or insulin formulation that provides a short term rapid release insulin and delivers an effective amount of insulin to a patient to manage the patient's blood glucose fluctuations following a meal. Typical prandial insulins include rapid-acting insulin analogs, which have a pharmacokinetic profile that closely resembles prandial endogenous insulin.

As used herein, "insulin" refers to human or non-human, recombinant, purified or synthetic insulin or insulin analogs, unless otherwise specified.

As used herein, "human insulin" is the human peptide hormone secreted by the pancreas, whether isolated from a natural source or made by genetically altered microorganisms.

As used herein, "non-human insulin" is insulin from a non-human animal source, such as a pig or cow. Bovine and porcine insulins differ in several amino acids from human insulin, but are bioactive in humans.

As used herein, an "insulin analog" is an altered insulin, different from the insulin secreted by the pancreas, but still available to the body for performing the same or similar action as natural insulin. Through genetic engineering of the underlying DNA, the amino acid sequence of insulin can be changed to alter its absorption, distribution, metabolism, and excretion (ADME) characteristics. Examples include, but are not limited to, insulin lispro, insulin glargine, insulin aspart, insulin glulisine, insulin detemir. The insulin can also be modified chemically, for example, by acetylation.

As used herein, "human insulin analogs" are altered human insulin which is able to perform a similar action as human insulin.

As used herein, an "excipient" is an inactive substance used as a carrier, to control release, increase isotonicity or aid the process by which a product is manufactured. In such cases, the insulin is dissolved or mixed with an excipient.

As used herin, a "precipitating agent" refers to a chemical that enhances the formation of an insulin microprecipitate, "seeds" an insulin precipitate, or stabilizes the insulin precipitate once formed by reducing its solubility at physiological pH and 37 °C.

As used herin, a "buffer" refers to a chemical agent that is able to absorb a certain quantity of acid or base without undergoing a strong variation in pH.

As used herein, a "stabilizing agent" refer to an agent that physically and chemically stabilizes the insulin by preventing the formation of breakdown products reducing the potency of the insulin.

As used herein, a "suspending agent" refers to a substance added to a formulation to retard the sedimentation of suspended particles in liquids.

As used herein, "hydrogel" refers to a water soluble hydrophilic polymer which may or may not be cross linked.

As used herein, "microenvironment" refers to the volume *in vivo* in which the formulation is located at a given time. The glucose levels in the microenvironment are generally relevant following administration and as the formulation is diluted until the formulation is diluted up to 20 times it initial concentration.

### II. Formnlations

The sterile injection formulations of the present invention are solutions that contain insulin, a glucose oxidase and peroxidase, one or more excipients, and optionally one or more thickening agents. The pH of the formulation prior to injection is below the isoelectric point of the insulin in the formulation. Following administration to a patient, the rate and amount of insulin released from the formulation and into the patient's systemic circulation is a function of the patient's blood glucose levels. The pH of the formulation prior to injection typically ranges from 3.5 and 5.5. The selection of insulin and oxidizing agent and the concentration of both the insulin and oxidizing agent, all effect the pharmacokinetic and pharmakodynamic (PK-PD) profile of the formulation. While many combinations are able to release sufficient amounts of insulin to a patient to achieve safe blood glucose levels, the preferred embodiment is selected based on its PK-PD profile, physiochemical characteristics, dosage form, and safety considerations.

The formulation contains any insulin. In one preferred embodiment, the insulin is a less soluble insulin.

### A. Insulin

Any insulin may be included in the formulation. Typically the formulation contains from 5 to 1,000U of insulin/ml of formulation, preferably 100U of insulin/ml of formulation, typically, the formulation contains greater than 20U of insulin/ml of the formulation.

### a. Less soluble Insulin

In one embodiment, the insulin is a less soluble insulin. The formulation contains any insulin that is less soluble than recombinant human insulin in extracellular fluid, such as Earle's balanced salt solution E2888 (Sigma Aldrich) at physiological pH (6.2-7.4) and body temperature (*e.g*. 37° C).

When the formulation is at pH 7 at room temperature or body temperature, the insulin is typically precipitated in the diluent and the formulation is in the form of a suspension. Suitable less soluble insulins that form a suspension include insulin glargine, NPH insulin, LENTE® insulin (*e.g.* (Humulin® L and Novolin® L) ULTRALENTE® insulin (*e.g*. Humulin® U), and protamine zinc insulin.

In one embodiment, the insulin in the formulation is insulin glargine (*e.g.* LANTUS® from Sanofi Aventis). Insulin glargine is a recombinant human insulin analog that differs from human insulin by having a glycine instead of asparagine at position 21 and two arginines added to the carboxy-terminus of the beta-chain. LANTUS® consists of insulin glargine dissolved in a clear aqueous fluid (100 IU, 3.6378 mg insulin glargine, 30 micrograms zinc, 2.7 mg m-cresol, 20 mg glycerol 85%, and water to 1 ml).

When forming the formulation described herein, the pH of LANTUS® is adjusted with an appropriate acid, such as HCl, to 4.0 and glucose oxidase and peroxidase are added to form the formulation. The pH of the formulation typically rises with the addition of the oxidizing agent and/or enzyme and reducing agent and/or enzyme and then is adjusted to 4.0 prior to injection. Following injection, small amounts of insulin glargine are released into the body continuously in response to the patient's blood glocuse levels, giving a basal release profile. The formulation slowly and continuously releases insulin for a prolonged period of time, such as from twelve to twenty-four hours following injection, preferably from 16 hours to 36 hours following injection.

### b. Other Insulins

In another embodiment, the formulation contains an insulin that typically has the same or a similar solubility in extracellular fluid, such as Earle's balanced salt solution E2888 (Sigma Aldrich), at physiological pH (6.2-7.4) and body temperature (*e.g*. 37° C) to the solubility of recombinant human insulin in the same conditions. In this embodiment, the formulation further comprises one or more components to modify the solubility of the insulin so that it is a less soluble insulin.

In this embodiment, the formulation is designed to release insulin at a rate that corresponds with a non-basal release profile following administration to a patient.

The insulin in the formulation may be human insulin, recombinant human insulin, insulin from a non-human animal source (*e.g*. bovine, porcine) or any other insulin, including insulin analogs that are soluble at a pH below physiological pH at 37 °C. The formulation is stabilized with a stabilizing agent, such as a source of zinc ions, and precipitation is initiated either by passage through the isoelectric point, or by addition of precipitating agents (*e.g*. arginine, histidine) that alter the solubility of insulin at neutral pH and 37 °C. In one embodiment the insulin is recombinant human insulin, and the formulation has a pH prior to injection ranging from 3.5 to 5.5, preferably from 3.8 to 4.2. In this embodiment, the formulation is designed to release insulin at a rate that corresponds with a regular human insulin release profile.

In one embodiment, the formulation is designed to release insulin at a rate that corresponds with a prandial release profile following administration to a patient. In this embodiment, the insulin in the formulation is typically a prandial insulin, such as an insulin analog of recombinant human insulin, which include, but are not limited to, insulin lispro, insulin glulisine, insulin aspart, or insulin detemir. Prandial insulins are rapidly absorbed into the systemic circulation and have a more rapid insulin peak (typically the peak in a graph of amount of insulin released into systemic administration over time occurs approximately 45 to 90 minutes after administration) than regular human insulin. A prandial insulin can be suspended with an oxidizing agent and/or an enzyme, such as GOD and POD, at a pH slightly below the pI of the particular insulin. Following administration of these formulations to a patient, the presence of elevated glucose levels would trigger the insulin release by reducing the pH of the microenvironment due to generation of gluconic acid from glucose. The lower microenvironmental pH increases the solubility of the insulin precipitate, enhancing the absorption of insulin into the systemic circulation. These formulations would be particularly suitable for regulating the release of insulin at the desired time in order to manage a patient's blood glucose levels following a meal. It is expected that the peak of insulin released from the formulation over time will be higher than the peak of insulin released from the same formulation containing the same insulin in the absence of glucose oxidase. Thus, the formulations described herein with a prandial release profile can release more insulin following a meal than the current prandial insulin formulations. These formulations are particularly useful at regulating a patient's blood glucose levels following a meal and preventing hyperglycemia, particularly in cases when a patient's blood glucose level is higher than the patient expected.

The insulin formulation can be made using any of the above mentioned insulins or a combination thereof and by combining it with GOD and POD.

### 1. Insulin Stabilizing agents

Stabilizing agents are included in the formulation specifically to stabilize insulin as a hexamer in solution. In the preferred embodiment, the stabilizing agent is zinc. This may be in the form of zinc acetate, zinc oxide, zinc citrate, zinc carbonate, zinc sulfate, or zinc chloride. In the preferred embodiment, zinc chloride is provided in the insulin solution at a concentration range of 0.1 to 10 mg/mL, preferably 2.5 mg/mL.

### 2. Precipitating agents,

Precipitating agents are added to enhance the formation of the insulin precipitate by either hastening the precipitate formation, and/or stabilizing the precipitate by reducing its solubility. These may be buffering agents, charged amino acids, precipitation seeding agents, and precipitation stabilization agents.

As the pH is increased from pH 4, towards physiological pH (7-7.5, typically 7.2-7.4), insulin transitions through its isoelectric point (pI). The amount or form may be increased or the form of the precipitate may be altered by increasing the residence time of the insulin at approximately its pI. This may be achieved by adding a buffering agent to the insulin formulation that is specifically selected for sufficient buffering capacity in the range of insulin's pI. Buffering agents include acetate, citrate, phosphate, carbonate, and barbital.

In the preferred embodiment, sodium acetate is used at a concentration ranging from 0.2 to 20mg/mL, preferably from 1 to 10 mg/mL, most preferably 5 mg/mL.

Addition of a charged molecule can enhance self-association of the insulin molecules. Examples of charged molecules include arginine, histidine, lysine and gluconate. A representative concentration of histidine ranges from 0.005 to 10 mg/mL, and preferably from 0.5 to 2 mg/ml.

Precipitation "seeding" agents may be a solid nanoparticle or a molecule that precipitate at or near the pI of the insulin that can thereby act as a nucleation site for the insulin to condense on. Examples of nanoparticles are Au₁₁ (present in the formulation in a concentration range from 24 to 2400 ng/ml, preferably 240 ng/ml,) and C₆₀ (present in the formulation in a concentration range from 75 to 7500 ng/ml, preferably 750 ng/mL). An example of a molecule that precipitates near the pI af insuline is cysteine with a pI of 5.0. An appropriate concentration of cysteine in the formulation ranges from 1.2 to 120 nM, and preferably is 12 nM.

Precipitation stabilizing agents are added to stabilize the newly formed precipitate, by reducing the solubility of the insulin at physiological pH. Precipitation stabilization agents include zinc chloride, calcium chloride and other divalent ions used at non-toxic levels (range 0.1-10 mg/ml, preferred 2.5 mg).

These precipitation agents may be used individually or combined to modify the pharmacokinetics of insulin precipitation and solubilization following injection. Typically these precipitation agents are added so that all of the insulin is solubilized within 8 to 24 hours following administration. The formulation is designed to create the best conditions for precipitation post injection, to leading to a stable micro-precipitate. The choice of agents may be dependent on the intended duration of the formulation (*e.g*. typically the formulation is intended to release insulin for 8 to 24 hours following injection, preferably for 12 to 24 hours following injection).

### B. Oxidation and Reduction Agents

The formulation contains an oxidizing agent or enzyme that oxidizes glucose. The formulation also contains a reducing agent or enzyme that reduces hydrogen peroxide. These oxidizing and reducing agents or enzymes change the pH of the microenvironment of the formulation in the presence of glucose.

The oxidizing enzyme is glucose oxidase. Glucose oxidase (GOD) converts glucose molecules to gluconic acid. As the concentration of glucose in the tissue rises, GOD oxidizes glucose to gluconic acid and hydrogen peroxide. During this oxidation process, hydrogen ions are generated, resulting in a lower pH in the formulation's microenvironment. The lower microenvironmental pH increases the solubility of the insulin precipitate, enhancing the absorption of insulin into the systemic circulation.

The reducing enzyme is peroxidase (POD) (also known as catalase). POD breaks the hydrogen peroxide produced from glucose oxidation reaction into water and oxygen, providing and/or maintaining the oxygen supply for the glucose oxidation reaction. It also eliminates unwanted hydrogen peroxide from the local tissue.

The formulation typically contains from 0.5 to 500 mg of GOD/ml of formulation and preferably contains 24 mg of GOD/ml of formulation. The formulation also typically contains from 1 to 500 µL of POD/mL of formulation, and preferably contains 30µL of POD/mL of formulation.

### C. Thickening Agents or gels

Optionally, the formulation contains a thickening agent or hydrogel. The thickening agent or hydrogel may serve to localize the formulation at the injection site following administration. The thickening agent or hydrogel may be present in an effective amount to reduce the diffusion rate of insulin out of the formulation compared to the diffusion rate of insulin out of the same formulation in the absence of the thickening agent or hydrogel. The thickening agent or gel must be biologically compatible. In the preferred embodiment the hydrogel or thickening agent is a synthetic polymer or a biopolymer, with the proviso that the thickening agent is not a chitosan-glycerol phosphate hydrogel, such as described in Kashyap, et al., Biomaterials, 28:2051-2060-2161 (2007) and Chenite, et al., Biomaterials, 21:2155-2161 (2000).

### D. Diluent

Typically the insulin is dissolved or dispersed in a diluent to provide the insulin in a liquid form. Suitable diluents include, but are not limited to, water, buffered aqueous solutions, vegetable or inert oils for injection organic hydrophilic diluents, such as monovalent alcohols, and low molecular weight glycols and polyols (*e*.*g*. propylene glycol, polypropylene glycol, glycerol, and butylene glycol).

Typically the diluent also serves as a carrier for the insulin formulation.

The diluent typically contains one or more excipients. Examples of excipients in a typical diluent for an injectable formulation include isotonic salts, preservatives, and optionally a buffering agent.

In the preferred embodiment, the diluent contains saline. In a further preferred embodiment, the diluent also contains one or more solubilizing agents and, optionally contains a thickening agent.

### E. Excipient and Carriers

In some embodiments, in addition to the diluent, the insulin may be combined with one or more pharmaceutically acceptable carriers to form the formulation for administration. In these embodiments, the diluent has a different composition than the carrier. In other embodiments, the diluent has the same composition as the carrier. In yet other embodiments, the diluent also serves as the carrier for the formulation.

As would be appreciated by one of skill in this art, the carriers must be suitable for administration by injection and is further selected based on the location of the target issue for administration of the formulation and the time course of delivery of the drug, such as sustained release, immediate release, basal release profile or non-basal release profile.

As used herein, the term "pharmaceutically acceptable carrier" means a non-toxic, semi-solid or liquid filler, or diluent.. Remington's Pharmaceutical Sciences Ed. by Gennaro, Mack Publishing, Easton, Pa., 1995 discloses various carriers used in formulating pharmaceutical compositions and known techniques for the preparation thereof.

Suitable excipients include surfactants, emulsifiers, emulsion stabilizers, anti-oxidants, emollients, humectants, suspending agents, thickening agents, occlusive agents, preservatives, stabilizing agents, pH modifying agents, solubilizing agents, solvents, colorants, penetration enhancers, isotonicity providing agents and other excipients.

### i. Emulsifiers

Suitable emulsifiers include, but are not limited to, straight chain or branched fatty acids, polyoxyethylene sorbitan fatty acid esters, sorbitan fatty acid esters, propylene glycol stearate, glyceryl stearate, polyethylene glycol, fatty alcohols, polymeric ethylene oxide-propylene oxide block copolymers, and combinations thereof.

### ii. Surfactants

Surfactants are wetting agents that lower the surface tension of a liquid, allowing easier spreading, and lower the interfacial tension between two liquids.

Suitable surfactants that may be included in the formulation include, but are not limited to, anionic surfactants, non-ionic surfactants, cationic surfactants, and amphoteric surfactants. Examples of anionic surfactants include, but are not limited to, ammonium lauryl sulfate, sodium lauryl sulfate, ammonium laureth sulfate, sodium laureth sulfate, alkyl glyceryl ether sulfonate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium and ammonium salts of coconut alkyl triethylene glycol ether sulfate; tallow alkyl triethylene glycol ether sulfate, tallow alkyl hexaoxyethylene sulfate, disodium N-octadecylsulfosuccinnate, disodium lauryl sulfosuccinate, diammonium lauryl sulfosuccinate, tetrasodium N-(1,2-dicarboxyethyl)-N-octadecylsulf osuccinnate, diamyl ester of sodium sulfosuccinic acid, dihexyl ester of sodium sulfosuccinic acid, dioctyl esters of sodium sulfosuccinic acid, docusate sodium, and combinations thereof.

Examples of nonionic surfactants include, but are not limited to, polyoxyethylene fatty acid esters, sorbitan esters, cetyl octanoate, cocamide DEA, cocamide MEA, cocamido propyl dimethyl amine oxide, coconut fatty acid diethanol amide, coconut fatty acid monoethanol amide, diglyceryl diisostearate, diglyceryl monoisostearate, diglyceryl monolaurate, diglyceryl monooleate, ethylene glycol distearate, ethylene glycol monostearate, ethoxylated castor oil, glyceryl monoisostearate, glyceryl monolaurate, glyceryl monomyristate, glyceryl monooleate, glyceryl monostearate, glyceryl tricaprylate/caprate, glyceryl triisostearate, glyceryl trioleate, glycol distearate, glycol monostearate, isooctyl stearate, lauramide DEA, lauric acid diethanol amide, lauric acid monoethanol amide, lauric/myristic acid diethanol amide, lauryl dimethyl amine oxide, lauryl/ myristyl amide DEA, lauryl/myristyl dimethyl amine oxide, methyl gluceth, methyl glucose sesquistearate, oleamide DEA, PEG-distearate, polyoxyethylene butyl ether, polyoxyethylene cetyl ether, polyoxyethylene lauryl amine, polyoxyethylene lauryl ester, polyoxyethylene lauryl ether, polyoxyethylene nonylphenyl ether, polyoxyethylene octyl ether, polyoxyethylene octylphenyl ether, polyoxyethylene oleyl amine, polyoxyethyelen oleyl cetyl ether, polyoxyethylene oleyl ester, polyoxyethylene oleyl ether, polyoxyethylene stearyl amine, polyoxyethylene stearyl ester, polyoxyethylene stearyl ether, polyoxyethylene tallow amine, polyoxyethylene tridecyl ether, propylene glycol monostearate, sorbitan monolaurate, sorbitan monooleate, sorbitan monopalmitate, sorbitan monostearate, sorbitan sesquioleate, sorbitan trioleate, stearamide DEA, stearic acid diethanol amide, stearic acid monoethanol amide, laureth-4, and combinations thereof.

Examples of amphoteric surfactants include, but are not limited to, sodium N-dodecyl-β-alanine, sodium N-lauryl-β-iminodipropionate, myristoamphoacetate, lauryl betaine, lauryl sulfobetaine, sodium 3-dodecyl-aminopropionate, sodium 3-dodecylaminopropane sulfonate, sodium lauroamphoacetate, cocodimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyeth- yl betaine, oleamidopropyl betaine, coco dimethyl sulfopropyl betaine, stearyl dimethyl sulfopropyl betaine, lauryl dimethyl sulfoethyl betaine, lauryl bis-(2-hydroxyethyl) sulfopropyl betaine, and combinations thereof.

Examples of cationic surfactants include, but are not limited to, behenyl trimethyl ammonium chloride (also known as "Behentrimonium Chloride"), bis(acyloxyethyl) hydroxyethyl methyl ammonium methosulfate, cetrimonium bromide, cetrimonium chloride, cetyl trimethyl ammonium chloride, cocamido propylamine oxide, distearyl dimethyl ammonium chloride, ditallowdimonium chloride, guar hydroxypropyltrimonium chloride, lauralkonium chloride, lauryl dimethylamine oxide, lauryl dimethylbenzyl ammonium chloride, lauryl polyoxyethylene dimethylamine oxide, lauryl trimethyl ammonium chloride, lautrimonium chloride, methyl-1-oleyl amide ethyl-2-oleyl imidazolinium methyl sulfate, picolin benzyl ammonium chloride, polyquaternium, stearalkonium chloride, stearyl dimethylbenzyl ammonium chloride, stearyl trimethyl ammonium chloride, trimethylglycine, and combinations thereof.

### iii. Suspending Agents

Suitable suspending agents include, but are not limited to, alginic acid, bentonite, carbomer, carboxymethylcellulose and salts thereof, colloidal oatmeal, hydroxyethylcellulose, hydroxypropylcellulose, microcrystalline cellulose, colloidal silicon dioxide, dextrin, gelatin, guar gum, xanthan gum, kaolin, magnesium aluminum silicate, maltitol, triglycerides, methylcellulose, polyoxyethylene fatty acid esters, polyvinylpyrrolidone, propylene glycol alginate, sodium alginate, chitosan, collagen, sorbitan fatty acid esters, tragacanth, and combinations thereof.

### iv. Antioxidants

Suitable antioxidants include, but are not limited to, butylated hydroxytoluene, alpha tocopherol, ascorbic acid, fumaric acid, malic acid, butylated hydroxyanisole, propyl gallate, sodium ascorbate, sodium metabisulfite, ascorbyl palmitate, ascorbyl acetate, ascorbyl phosphate, Vitamin A, folic acid, flavons or flavonoids, histidine, glycine, tyrosine, tryptophan, carotenoids, carotenes, alpha-Carotene, beta-Carotene, uric acid, pharmaceutically acceptable salts thereof, derivatives thereof, and combinations thereof.

### v. Humectants

Suitable humectants include, but are not limited to, glycerin, butylene glycol, propylene glycol, sorbitol, triacetin, and combinations thereof

### vi. pH Modifying Agents

The compositions described herein may further contain sufficient amounts of at least one pH modifier to ensure that the composition has a final pH within a physiologically acceptable range, such as from about 3.5 to about 7.4. Suitable pH modifying agents include, but are not limited to, sodium hydroxide, citric acid, hydrochloric acid, acetic acid, phosphoric acid, succinic acid, sodium hydroxide, potassium hydroxide, ammonium hydroxide, magnesium oxide, calcium carbonate, magnesium carbonate, magnesium aluminum silicates, malic acid, potassium citrate, sodium citrate, sodium phosphate, lactic acid, gluconic acid, tartaric acid, 1,2,3,4-butane tetracarboxylic acid, fumaric acid, diethanolamine, monoethanolamine, sodium carbonate, sodium bicarbonate, triethanolamine, sodium acetate and combinations thereof.

### vii. Preservatives

Preservatives can be included in the formulation in an effective amount to prevent the growth of fungi and other microorganisms. Suitable preservatives include, but are not limited to, benzoic acid, butylparaben, ethyl paraben, methyl paraben, propylparaben, sodium benzoate, sodium propionate, benzalkonium chloride, benzethonium chloride, benzyl alcohol, cetypyridinium chloride, chlorobutanol, phenol, phenylethyl alcohol, thimerosal, metacresol and combinations thereof.

### F. Dosage Forms

The formulation of the present invention is a sterile injectable solution. The initial pH of the formulation is below the isoelectric point for the particular insulin in the formulation. In one embodiment, the initial pH of the formulation ranges from 3.5 to 5.5, preferably the initial pH of the formulation ranges from 3.8 to 4.2.

The formulation is typically administered parenterally such as but not limited to subcutanteously, intramuscularly, or intradermally. In preferred embodiment, the formulation is injected subcutaneously.

The ability of a particular insulin formulation to release insulin as a function of glucose levels can be assessed by a suitable experiment, such as but not limited to *in vitro* glucose challenge experiments, dissolution experiments with release media containing glucose levels at 150mg/dl or above, or in a diabetic animal model, such as but not limited to diabetic swine, diabetic mice, diabetic rat, and diabetic dog.

The insulin formulations are preferably formulated in dosage unit form for ease of administration and uniformity of dosage. The expression "dosage unit form" as used herein refers to a physically discrete unit of conjugate appropriate for the patient to be treated. It will be understood, however, that the total daily usage of the compositions of the present invention will be decided by the attending physician within the scope of sound medical judgment. For any conjugate, the therapeutically effective dose can be estimated initially either in cell culture assays or in animal models, usually mice, rabbits, dogs, or pigs.

Sterile injectable preparations may be formulated as known in the art. The sterile injectable preparation may be a solution, suspension, or emulsion in a nontoxic parenterally acceptable diluent or solvent. Among the acceptable vehicles and solvents that may be employed are water, Ringer's solution, U.S.P. and isotonic sodium chloride solution. In addition, sterile, fixed oils can be employed as a solvent or suspending medium. For this purpose any bland fixed oil can be employed including synthetic mono- or diglycerides, peanut oil, sesame oil or any other vegetable oils. In addition, fatty acids such as oleic acid can be used in the preparation of injectable formulations. The injectable formulations can be sterilized, for example; by filtration through a bacteria-retaining filter, or by incorporating sterilizing agents in the form of sterile solid compositions which can be dissolved or dispersed in sterile water or other sterile injectable medium prior to use.

### III. Methods of making the formulations

In one embodiment, the formulation is formed by mixing powdered components of the formulation, such as glucose oxidase and peroxidase and any excipients in powdered form, such as pH modifying agents, polymers, thickening agents or hydrogel forming materials, which initially are in powdered form, suspending agents, surfactants, antioxidants, and preservatives and combinations thereof, with a liquid diluent that contains the insulin.

In the preferred embodiment, the insulin formulation is made by combining all constituents into the diluent, and adjusting to a final pH to make a suspension. The suspension is sterilized and filled in a vial suitable for multiple injection dosing.

In one embodiment, for formulations that contain LANTUS®, the pH of LANTUS® is adjusted with an appropriate acid, such as HCl, to 4.0 and glucose oxidase and peroxidase are added to form the formulation. The pH of the formulation typically rises with the addition of the oxidizing agent and/or enzyme and reducing agent and/or enzyme and then is adjusted to 4.0 prior to injection.

Optionally, the insulin and powdered components are provided in lyophilized form in one compartment of a kit, such as a vial, and the liquid components, *i.e*. the diluent, is provided in a second compartment, such as a second vial. Optionally, one or more excipients are present in one or both vials, as appropriate to adjust pH, and stabilize and buffer the formulation.

Pharmaceutical compositions may also be formulated in any other conventional manner using one or more physiologically acceptable carriers comprising excipients and auxiliaries which facilitate processing of the insulin into preparations which can be used pharmaceutically. Formulation of drugs is discussed in, for example, Hoover, John E., Remington's Pharmaceutical Sciences, Mack Publishing Co., Easton, Pennsylvania (1975), and Liberman, H.A. and Lachman, L., Eds., Pharmaceutical Dosage Forms, Marcel Decker, New York, N.Y. (1980). Proper formulation is dependent upon the route of administration chosen.

### IV. Methods of using the formulations

The formulations may be administered by subcutaneous, intradermal, or intramuscular injection. Preferably, the formulation is administered via subcutaneous injection.

For ease of injection, the formulations are preferably administered as a liquid, preferably in the form of an injectable suspension. Optionally, the viscosity of the formulation may increase *in vivo* to form a gel. In one embodiment, the formulation is designed to release insulin into systemic circulation over time with a basal release profile following injection in a patient. In another embodiment, the formulation is designed to release insulin into systemic circulation over time with a non-basal release profile following injection in a patient. Exemplary non-basal release profiles include a regular human insulin release profile and a prandial release profile. In one embodiment the formulation is designed to release insulin into systemic circulation over time with a regular human insulin release profile following injection in a patient. In another embodiment, the formulation is designed to release insulin into systemic circulation over time with a prandial release profile following injection in a patient.

As the patient's blood glucose levels rise, the glucose is oxidized by the oxidizing agent, such as GOD, resulting in production of hydrogen ions from gluconic acid in the micro environment of the formulation at injection site. The increase in hydrogen ion production will lower the pH of the microenvironment. The lower microenvironmental pH increases the solubility of the insulin precipitate, enhancing the absorption of insulin into the systemic circulation. Availability of insulin in systemic circulation leads to a decrease in blood glucose levels. Following this, the reaction that converts glucose to gluconic acid slows down. Thus fewer hydrogen ions are produced, and the pH of the microenvironment rises. This returns insulin to its less soluble state in the absence of high blood glucose levels.

In a preferred embodiment, the insulin formulation is administered to patients who are not fully insulin dependent. In one embodiment, the formulation provides a sufficient amount of insulin to the patient during the day so that the patient does not require additional insulin-containing formulations to maintain his/her blood glucose levels within a safe range. The patient is typically not fully insulin dependent.

In another embodiment, the formulation is administered to a patient who is receiving intensive insulin therapy as one of the insulin-containing formulations administered to the patient during the day. Preferably the formulation delivers insulin to the patient with a basal release profile.

Unless defined otherwise, all technical and scientific terms used herein have the same meanings as commonly understood by one of skill in the art to which the disclosed invention belongs.

Those skilled in the art will recognize, or be able to ascertain using no more than routine experimentation, many equivalents to the specific embodiments of the invention described herein. Such equivalents are intended to be encompassed by the claims.

### Examples

### Example 1: In vitro response of system to varying glucose

Precipitated insulin formulations were placed in the upper well of a new transwell device and placed in a 6-well plastic plate. Solutions containing saline with and without glucose were added to the receiver side of the transwell plates. Samples were taken from the receiver compartment of the transwell plate, and media was replaced to maintain a constant volume during the experiment. Insulin concentrations were determined by HPLC.

### Materials

Glucose oxidase, from A. niger, Sigma
Peroxidase from A. niger, Sigma
Insulin Glargine solution 100 U/ml, Sanofi Aventis
Glucose, Fisher Scientific
Dulbecco's phosphate buffer saline (DPBS), Gibco
Saline 0.9% w/v
Transwell cell culture inserts and six well plates, Falcon

### Methods

A Smart basal insulin formulation was prepared as follows. 48 mg of GOD and 60µl of POD were added to the Insulin glargine solution. The solution turned cloudy upon addition of the enzymes, GOD and POD. The pH of this solution was measured and then adjusted to 4.

### HPLC Assay

The amount of insulin released in the presence and absence of glucose was determined using reverse phase chromatography on a C-18 column with a mobile phase composition of 71 ml Water: 20 ml Acetonitrile: 9 ml Tetrahydrofuran and 0.1 % TFA. The HPLC acquisition parameters were flow rate 1.0 ml/min, Sample Temperature 5°C and Column Temperature 40°C, 210 nm detection.

### Experiment

Two sets of glucose samples were made, Set 1 and 2. Set 1 had 300mg/dl effective glucose in the formulation, and Set 2 had no glucose. In Set 1, 200µl of glucose DPBS was added so that the effective glucose concentration in the insert was 300 mg/dl glucose. The receiver well contained 1.5ml of 300 mg/dl glucose-saline.

In Set 2, 200µl of blank DPBS was added to the top of the cell insert and 1.5 mL to the receiver well.

One ml of the Smart basal formulation was placed in the cell culture inserts without any cells for each Set.

Aliquots of 500µl were sampled at 3h and 6h from each receiver well. The volume was replaced each time an aliquot was removed with the respective receiver solution. The amount of insulin released under different glucose conditions was compared between the glucose (Set 1) and no glucose (Set 2) sets. Effect of glucose concentration on the amount of insulin released was also studied as described above. The pH of each receiver well was also measured at 3 hours and 6 hours using a commercial glucose strip method (OneTouch® by LifeScan, Inc.).

### Results

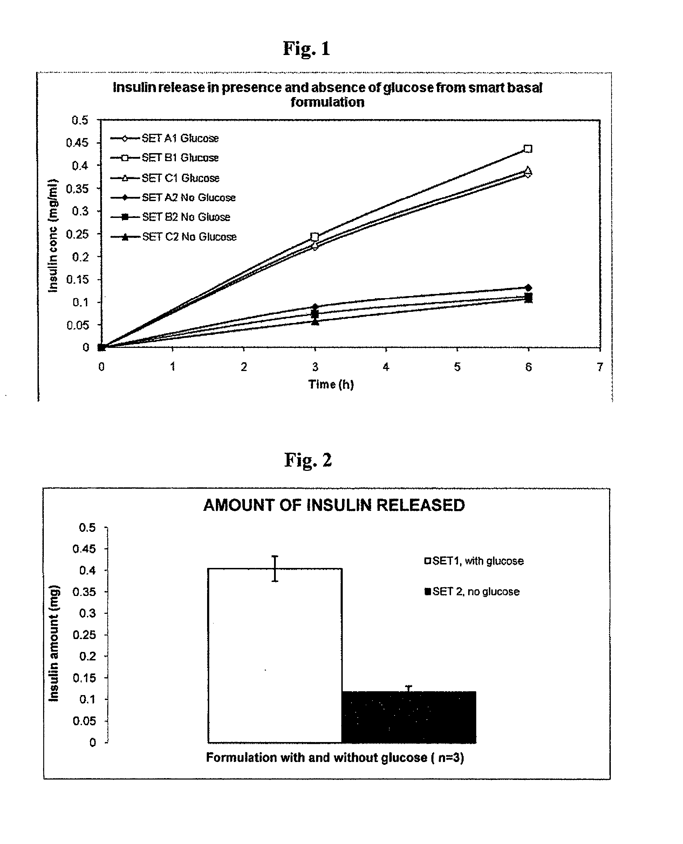

Figure 1 and 2 show that amount of insulin released by the Smart basal formulation in presence of glucose was higher than in absence of glucose. This confirms that the formulation is responsive to the glucose in its environment.

It appears that the inclusion of GOD in the system and subsequent generation of gluconic acid, alters the release pattern and/ or amount of insulin released when compared to the control without glucose.

Gluconic acid caused the pH of the inserts go down from pH 4 to approximately pH 3.5 and thereby solubilized precipitated insulin glargine. The increased solubility led to higher insulin amounts recovered in the release medium (*see* Figures 1 and 2). However, in the control set, the pH of formulation remained unchanged at pH 4.

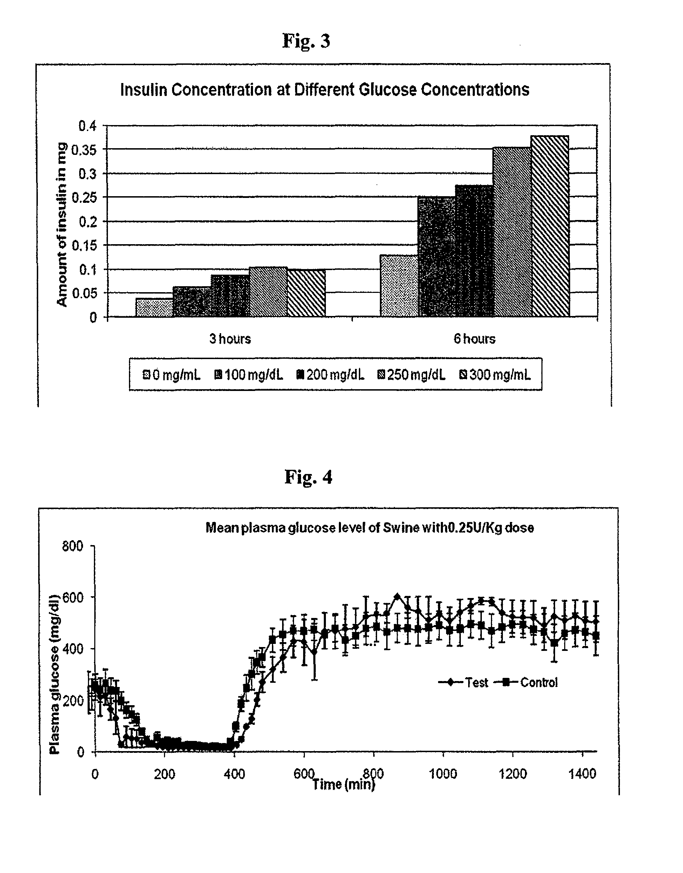

It was also seen that the amount of insulin released from the Smart basal formulation was dependent upon the glucose concentration in the environment. In presence of higher glucose concentration, higher insulin amounts were released compared to insulin exposed to a lower glucose concentration. The release of insulin is also dependent on the gluconic acid generated from the available glucose concentration (Figure 3).

### Example 2: Administration of an Insulin dose of 0.25U/kg to Diabetic Swine, comparing LANTUS® to a Smart basal insulin formulation

### Materials and Methods

Six (6) Diabetic swine were fasted overnight. Morning glucose levels were high and were used as the starting point for the comparison for the Insulin glargine alone (control) with the Smart basal formulation described in Example 1 (test formulation)). Three swine were tested with each formulation. The dose administered subcutaneously to each pig was 0.25U/kg. Following administration of the formulation, the pigs were monitored and fed at 360 minutes. Plasma glucose levels were determined every fifteen minutes via a commercial glucose strip method (OneTouch® by LifeScan, Inc.).

### Results

Mean results of three swine are shown in Figure 4, plus or minus standard error of the mean for each formulation. It appears that the test group responded to the elevated glucose levels both initially and upon second feeding more rapidly than the control group, indicating the glucose oxidase/peroxidase enhanced the release of insulin glargine.

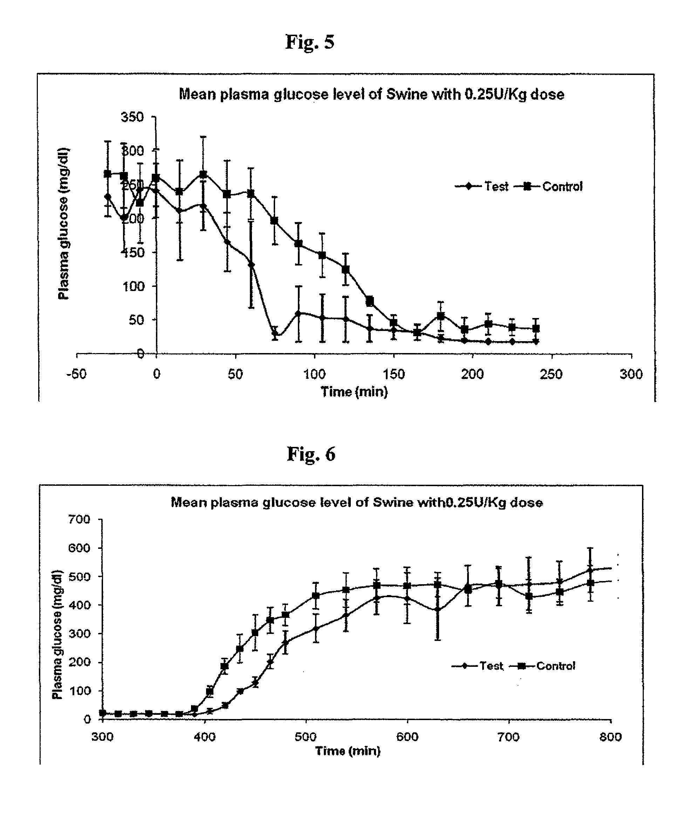

Figures 5 and 6 highlight the differences in plasma glucose levels (PGL) between the test and control group, initially and post feeding. Following subcutaneous administration, the smart basal insulin formulation decreased the PGL of the test group faster than LANTUS® decreased the PGL of the control group. This can be attributed to the glucose responsiveness of the formulation contributed by the GOD acting as a glucose sensor.

After the rapid decrease in PGL in the test group, glucose levels of the test and control group overlapped during the time period ranging from approximately 180 minutes to 400 minutes, showing that the glucose sensor in the smart insulin formulation only acts on high glucose concentrations to convert glucose to gluconic acid. Thus, in the absence of high plasma glucose values, the amount and rate of insulin that was released from the test formulation had a basal profile, which was similar to the control formulation (*see* Figure 4).

After feeding at 360 minutes, the PGL of the swine increased quickly (*see* Figure 6). Because of the food intake, smart insulin system responded to the food and slowed the rate of glucose increase unlike in control group where higher glucose levels were seen during the defined time period of approximately 400 to 550 minutes. After approximately 12 hours, the test group had a higher mean PGL than the control group. One explanation for this difference, may be attributed to the faster consumption of the insulin from the test formulation due to glucose responsive release profile. Thus the smart basal insulin formulation tested showed that the magnitude of the bioavailability of the insulin is directly dependent on tissue glucose concentrations.

### Example 3: Administration of an Insulin dose of 0.45U/kg to Diabetic Swine under normal feeding conditions, comparing LANTUS® to a Smart basal insulin formulation

### Materials and Methods

Diabetic swine were given food and their maintenance insulin dose the evening prior to the test day. On the test day, animals were administered the test and control doses and then fed at 0h and 360 minutes. The control group received Insulin glargine alone, and the test group received the Smart basal formulation described in Example 1. The dose was 0.45U/Kg. Animals were administered dose and fed at t=0 minute and re-fed at 360 minutes. Plasma samples were collected and analyzed for plasma insulin and plasma glucose levels.

### Results

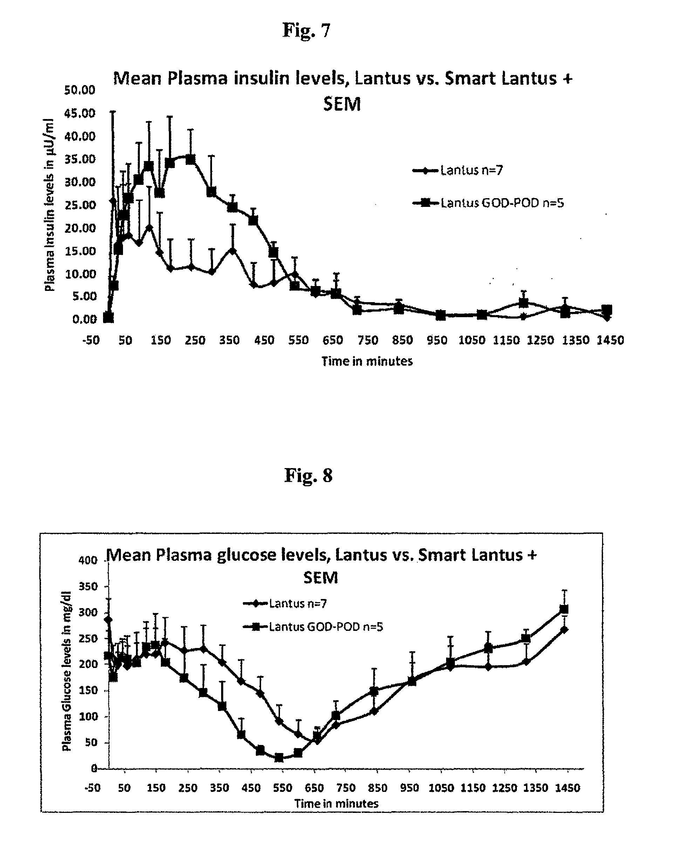

Comparative mean plasma insulin levels (µU/ml) with the standard error of the mean for the control (Mean of N=7) and test group (Mean of N=5) are shown in Figure 7. Comparative mean plasma glucose levels (mg/dl) with the standard error of the mean for the control (Mean of N=7) and test group (Mean of N=5) are shown in Figure 8.

As shown in Figures 7 and 8, it appears that under the normal food and dosing conditions, the test group responded more rapidly to high glucose conditions compared to the response of the control group. As seen from Figure 7, more insulin was released compared to the insulin released from the control in response to elevated glucose condition arising from multiple feedings. Figure 8 shows that there was faster pharmacodynamic response in the test group compared to the response in the control group. Thus, the smart basal insulin formulation brought the plasma glucose levels down at a faster rate than the control group.

These examples indicate that the smart basal insulin system is responsive to blood glucose concentrations and releases insulin, based on a patient's blood glucose levels.

## Claims

1. A sterile injectable formulation, which is a solution, comprising insulin, a diluent suitable for injection, glucose oxidase and peroxidase,
wherein the pH of the formulation is below the isoelectric point of the insulin,
wherein the solubility of the insulin increases or decreases based on the pH of the microenvironment of the formulation, and
wherein insulin is released from the formulation as a function of a patient's tissue glucose levels.

2. The formulation of claim 1, wherein the insulin is insulin glargine.

3. The formulation of claim 1, wherein the insulin is recombinant human insulin.

4. The formulation of claim 1, wherein the insulin is an insulin analog of recombinant human insulin.

5. The formulation of claim 4, wherein the insulin analog is selected from the group consisting of insulin lispro, insulin glulisine, insulin aspart, and insulin detemir.

6. The formulation of claim 1, wherein the formulation has a pH ranging from 3.5 to 5.5.

7. The formulation of claim 2, wherein the formulation has a pH ranging from 3.5 and 5.5, preferably 3.8 to 4.2.

8. The formulation of claim 7, wherein the formulation further comprises a stabilizer, buffering agent and precipitating agent.

9. The formulation of any preceding claim for use in medicine.

10. The formulation of any preceding claim for use in a method of treating a patient with diabetes, the method comprising administering to the patient via injection a basal amount of said formulation.

11. The formulation of claim 10, wherein the formulation provides prolonged levels of insulin over a peroiod of time after administrtation of 10 to 24 hours and delivers an effective amount of insulin to a patient to manage the patient's normal dialy blood glucose fluctuations in the absence of a meal.

12. The formulation of any one of claims 1-9 for use in a method for regulating a patient's blood glucose levels, the method comprising administering to the patient via injection of said formulation, wherein the insulin is released from the formulation as a function of the patient's tissue glucose levels.

## Patentansprüche

1. Sterile injizierbare Formulierung, die eine Lösung ist, umfassend Insulin, ein für Injektionszwecke geeignetes Verdünnungsmittel, Glucoseoxidase und Peroxidase,
wobei der pH-Wert der Formulierung unter dem isoelektrischen Punkt des Insulins liegt,
wobei die Löslichkeit des Insulins auf der Basis des pH-Werts der Mikroumgebung der Formulierung zunimmt oder abnimmt und
wobei Insulin aus der Formulierung als Funktion der Glucosespiegel des Gewebes eines Patienten freigesetzt wird.

2. Formulierung nach Anspruch 1, wobei das Insulin Insulin Glargine ist.

3. Formulierung nach Anspruch 1, wobei das Insulin rekombinantes humanes Insulin ist.

4. Formulierung nach Anspruch 1, wobei das Insulin ein Insulinanalogon von rekombinantem humanem Insulin ist.

5. Formulierung nach Anspruch 4, wobei das Insulinanalogon aus der Gruppe von Insulin Lispro, Insulin Glulisine, Insulin Aspart und Insulin Detemir ausgewählt ist.

6. Formulierung nach Anspruch 1, wobei die Formulierung einen pH-Wert im Bereich von 3,5 bis 5,5 aufweist.

7. Formulierung nach Anspruch 2, wobei die Formulierung einen pH-Wert im Bereich von 3,5 bis 5,5, vorzugsweise 3,8 bis 4,2 aufweist.

8. Formulierung nach Anspruch 7, wobei die Formulierung ferner ein Stabilisierungsmittel, ein Puffermittel und ein Fällungsmittel umfasst.

9. Formulierung nach einem der vorhergehenden Ansprüche zur Verwendung in der Medizin.

10. Formulierung nach einem der vorhergehenden Ansprüche zur Verwendung in einem Verfahren zur Behandlung eines Patienten mit Diabetes, wobei das Verfahren das Verabreichen einer Basalmenge der Formulierung an den Patienten über eine Injektion umfasst.

11. Formulierung nach Anspruch 10, wobei die Formulierung verlängerte Insulinspiegel über einen Zeitraum von 12 bis 24 h nach der Verabreichung ergibt und einem Patienten eine wirksame Insulinmenge liefert, um die normalen täglichen Blutglucoseschwankungen eines Patienten bei Fehlen einer Mahlzeit zu behandeln.

12. Formulierung nach einem der Ansprüche 1-9 zur Verwendung in einem Verfahren zur Regelung der Blutglucosespiegel eines Patienten, wobei das Verfahren das Verabreichen der Formulierung an den Patienten über eine Injektion umfasst, wobei das Insulin aus der Formulierung als Funktion der Gewebeglucosespiegel des Patienten freigesetzt wird.

## Revendications

1. Formulation injectable stérile, qui est une solution, comprenant de l'insuline, un diluant approprié pour injection, du glucose, une oxydase et une peroxydase,
où le pH de la formulation est inférieur au point isoélectrique de l'insuline,
où la solubilité de l'insuline augmente ou diminue en fonction du pH du microenvironnement de la formulation, et
où l'insuline est libérée à partir de la formulation en fonction des taux tissulaires de glucose d'un patient.

2. Formulation selon la revendication 1, dans laquelle l'insuline est l'insuline glargine.

3. Formulation selon la revendication 1, dans laquelle l'insuline est de l'insuline humaine recombinante.

4. Formulation selon la revendication 1, dans laquelle l'insuline est un analogue insulinique de l'insuline humaine recombinante.

5. Formulation selon la revendication 4, dans laquelle l'analogue insulinique est choisi dans le groupe constitué par l'insuline lispro, l'insuline glulisine, l'insuline aspart, et l'insuline détémir.

6. Formulation selon la revendication 1, où la formulation a un pH situé dans la plage de 3,5 à 5,5.

7. Formulation selon la revendication 2, où la formulation a un pH situé dans la plage de 3,5 à 5,5, de préférence 3,8 à 4,2.

8. Formulation selon la revendication 7, où la formulation comprend en outre un stabilisant, un agent tampon et un agent de précipitation.

9. Formulation selon l'une quelconque des revendications précédentes, pour une utilisation en médecine.

10. Formulation selon l'une quelconque des revendications précédentes, pour une utilisation dans un procédé de traitement d'un patient souffrant de diabète, le procédé comprenant l'administration au patient par injection d'une quantité de base de ladite formulation.

11. Formulation selon la revendication 10, où la formulation fournit des taux prolongés d'insuline sur une période de temps après l'administration de 12 à 24 heures et délivre une quantité efficace d'insuline à un patient pour contrôler les fluctuations quotidiennes normales de la glycémie du patient en l'absence d'un repas.

12. Formulation selon l'une quelconque des revendications 1 à 9, pour une utilisation dans un procédé de régulation des taux de glycémie d'un patient, le procédé comprenant l'administration au patient par injection de ladite formulation, où l'insuline est libérée à partir de la formulation en fonction des taux tissulaires de glucose du patient.
